# EUROPEAN PATENT SPECIFICATION

(11) **EP 1 289 503 B1**
(45) Date of publication and mention of the grant of the patent: **27.06.2007**
(21) Application number: 01931897.1
(22) Date of filing: 22.05.2001
(51) Int. Cl.: A61K 9/107, A61K 47/14, A61K 47/44, A61K 31/05

(54) **O/W EMULSION**
O/W EMULSION
Emulsion o/w

(30) Priority: 25.05.2000 GB 0012597
(43) Date of publication of application: 12.03.2003
(73) Proprietor: AstraZeneca AB, 151 85 Södertälje (SE)
(72) Inventor: GOODALE, David, Buell, Wilmington, DE 19850-5437 (US)
(86) International application number: PCT/GB2001/002259
(87) International publication number: WO 2001/089474

(56) References cited:
- WO-A-00/10531
- WO-A-00/24376
- WO-A-95/20943

## Description

The invention relates to pharmaceutical compositions comprising a free radical scavenging sedative agent and their use in improving survival in certain patents. In particular, the present invention relates to 2,6-diisopropylphenol, known as propofol, and to pharmaceutical compositions containing propofol, and their use.

Propofol is an injectable anaesthetic which has hypnotic properties and can be used to induce and maintain general anaesthesia and for sedation, for example in Intensive Care Units. Propofol is a highly successful anaesthetic and is marketed under the trademark 'Diprivan' for use in treating humans and under the trademark 'Rapinovet' for veterinary use.

Injectable anaesthetics, such as propofol, are administered directly into the blood stream. This gives rise to a rapid onset of anaesthesia influenced almost entirely by the rate at which the anaesthetic agent crosses the blood-brain barrier. It is therefore necessary for the anaesthetic agent to have sufficient lipid solubility to be able to cross this barrier and depress the relevant mechanisms of the brain. However highly lipid soluble molecules are generally poorly soluble in water and thus are difficult to formulate for intravenous injection. In some cases it may be possible to obtain a water soluble salt of the anaesthetic agent which releases a lipid soluble free base in vivo. This is not possible in many cases and, despite considerable research, it did not prove to be feasible with propofol. Thus it was necessary to conduct very substantial research and development into the formulation of propofol in order to obtain pharmaceutical compositions for administration to warm-blooded animals including humans.

Once the anaesthetic properties of propofol were recognised, UK patent application no 13739/74 was filed, which was granted as United Kingdom Patent 1472793. Corresponding patents have been granted in the USA (USP 4056635, USP 4452817 and USP 4798846) and many other territories. UK 1472793 describes many particular Examples of injectable compositions containing propofol including Examples with various surfactants, various solubilising agents, additional solvents, additional constituents (selected from stabilisers, preservatives and antioxidants), buffering agents and tonicity modifiers. A substantial amount of work on formulations was performed and an oil-in-water emulsion using a vegetable oil (soy bean) was eventually selected for development.

The finding that adventitious extrinsic microbial contamination resulting from non-asceptic handling of the original formulation of 'Diprivan' could lead to 'clusters' of postoperative infection, initiated development of a modified formulation with a suitable additive present (the additive being capable of retarding the growth of common micro-organisms to not greater than 1 log increase (ie, 10 fold) in 24 hours following extrinsic contamination equivalent to 'touch contamination'). The development of a modified, edetate-containing formulation of propofol led to the filing and grant, inter alia, of UK Patent No. 2,298,789. Corresponding patents have been granted, for example, in the USA (USP 5,714,520; USP 5,731,355; USP 5,731,356; USP 5,908,869) and in other territories. The marketed modified formulation of 'Diprivan' contains 0.005% disodium edetate.

UK Patent No. 2,298,789 describes that a wide range of water-immiscible solvents can be used. Typically the water-immiscible solvent is a vegetable oil, for example soy bean, safflower, cottonseed, corn, sunflower, arachis, castor or olive oil. Preferably the vegetable oil is soy bean oil.

An increasing proportion of the usage of 'Diprivan' is in the sedation of seriously ill patients particularly in Intensive Care Units and the like. In the sedation of such seriously ill patients administration of 'Diprivan' is typically by means of infusion. Despite advances in critical care medicine the rate of survival of critically ill patient remains poor, and is quite variable, with typical mortality rates in the range 10% to 40%. Beyond the development of antibiotics and improvements in medical techniques (such as newer resuscitation techniques, for example ventilation and fluids administration) there has probably been little impact on survival rates in critical illness despite many years of medical research involving many drugs and treatment regimes. There is therfore a large unmet medical need for improving the rate of survival in critically ill patients.

The present applicants have considered extending the range of propofol formulations in order to give the anaesthetist a wider armamentarium from which to select an appropriate drug, in particular for treating seriously ill patients, and improving the survival of critically ill patients. In considering appropriate further formulations it is desirable to maintain the qualities that make 'Diprivan' of such merit and provide a formulation with acceptable chemical and physical stability and which is readily manipulable by the anaesthetist or Intensive Care Unit (ICU) specialist.

Lipids are important in many biological processes, and the water-immiscible solvent in which propofol is administered has been adapted in the present invention to provide a particularly beneficial propofol formulation, in particular for treating seriously ill patients. A balance needs to be achieved to satisfy many physical and chemical requirements as well as achieving a desirable biological lipid profile.

Accordingly, the present invention provides a sterile pharmaceutical composition for parenteral administration, which composition comprises an oil-in-water emulsion in which propofol dissolved in an omega-3 rich water-immiscible solvent, is emulsified with water and stabilised by means of a surfactant.

More particularly, the present invention provides a sterile pharmaceutical composition for parenteral administration, which composition comprises an oil-in-water emulsion in which propofol dissolved in an omega-3 rich water-immiscible solvent, is emulsified with water and stabilised by means of a surfactant, and which further comprises a metal ion chelating agent.

The invention further provides a method of producing a sedative or anaesthetic effect which comprises administration of a sterile pharmaceutical composition for parenteral administration, which composition comprises an oil-in-water emulsion in which propofol dissolved in an omega-3 rich water-immiscible solvent, is emulsified with water and stabilised by means of a surfactant.

Yet more particularly, the invention provides a method of producing a sedative or anaesthetic effect which comprises administration of a sterile pharmaceutical composition for parenteral administration, which composition comprises an oil-in-water emulsion in which propofol dissolved in an omega-3 water-immiscible solvent, is emulsified with water and stabilised by means of a surfactant, and which further comprises a metal ion chelating agent.

The invention further provides a method of improving survival in critically ill patients which comprises administration of a sterile pharmaceutical composition for parenteral administration, which composition comprises an oil-in-water emulsion in which propofol dissolved in an omega-3 rich water-immiscible solvent, is emulsified with water and stabilised by means of a surfactant.

Yet more particularly, the invention provides a method of improving survival in critically ill patients which comprises administration of a sterile pharmaceutical composition for parenteral administration, which composition comprises an oil-in-water emulsion in which propofol dissolved in an omega-3 water-immiscible solvent, is emulsified with water and stabilised by means of a surfactant, and which further comprises a metal ion chelating agent.

The invention further provides a sterile pharmaceutical composition for parenteral administration, which composition comprises an oil-in-water emulsion in which propofol dissolved in an omega-3 rich water-immiscible solvent, is emulsified with water and stabilised by means of a surfactant for use as a medicament, in particular for producing a sedative or anaesthetic effect.

In particular, the invention provides a sterile pharmaceutical composition for parenteral administration, which composition comprises an oil-in-water emulsion in which propofol dissolved in an omega-3 water-immiscible solvent, is emulsified with water and stabilised by means of a surfactant, and which further comprises a metal ion chelating agent, for use as a medicament, in particular for producing a sedative or anaesthetic effect.

The invention further provides a sterile pharmaceutical composition for parenteral administration, which composition comprises an oil-in-water emulsion in which propofol dissolved in an omega-3 rich water-immiscible solvent, is emulsified with water and stabilised by means of a surfactant for use as a medicament, in particular for improving survival in critically ill patients.

In particular, the invention provides a sterile pharmaceutical composition for parenteral administration, which composition comprises an oil-in-water emulsion in which propofol dissolved in an omega-3 water-immiscible solvent, is emulsified with water and stabilised by means of a surfactant, and which further comprises a metal ion chelating agent, for use as a medicament, in particular for improving survival in critically ill patients.

The invention further provides the use of a sterile pharmaceutical composition for parenteral administration, which composition comprises an oil-in-water emulsion in which propofol dissolved in an omega-3 water-immiscible solvent, is emulsified with water and stabilised by means of a surfactant, for the manufacture of a medicament for producing a sedative or anaesthetic effect.

The invention further provides the use of a sterile pharmaceutical composition for parenteral administration, which composition comprises an oil-in-water emulsion in which propofol dissolved in an omega-3 water-immiscible solvent, is emulsified with water and stabilised by means of a surfactant, and which further comprises a metal ion chelating agent, for the manufacture of a medicament for producing a sedative or anaesthetic effect.

The invention further provides the use of a sterile pharmaceutical composition for parenteral administration, which composition comprises an oil-in-water emulsion in which propofol dissolved in an omega-3 water-immiscible solvent, is emulsified with water and stabilised by means of a surfactant, for the manufacture of a medicament for improving survival in critically ill patients.

The invention further provides the use of a sterile pharmaceutical composition for parenteral administration, which composition comprises an oil-in-water emulsion in which propofol dissolved in an omega-3 water-immiscible solvent, is emulsified with water and stabilised by means of a surfactant, and which further comprises a metal ion chelating agent, for the manufacture of a medicament for improving survival in critically ill patients.

Without wishing to be constrained by theory, we believe that the beneficial effects of the present invention may result from the formulation components delivering lower levels of stress-related agents and/or an anti-oxidative effect and/or a beneficial immune function protection/suppression profile.
The benefit is believed to arise, in part, from the free radical scavenging/anti-oxidative effects of the sedative propofol. Thus, formulations containing other free radical scavenging/anti-oxidative sedatives may also show benefit. By a 'free radical scavenging sedative agent' we mean a sedative agent that also has the ability to scavenge free radicals in-vivo (with the ability to counteract free radical mediated oxidative stress processes). Apart from propofol, other such agents may include, for example, barbiturates such as phenobarbitol. The free radical scavenging sedative agent is preferably lipophilic in nature. Also included in the present invention are compositions comprising pro-drugs of propofol, which may be metabolised to provide propofol per se in-vivo.

Benefit is also believed to arise in part from the immune function protection/suppression profile produced by the omega-3 rich water immiscible solvent. Omega-3 oils possess immune activating properties, whilst omega-6 oils possess immune suppressive properties. The particular balance and immune function profile achieved by the present omega-3 rich based formulations is believed to be particularly beneficial.

Further benefit may also arise, in part, from the presence of a metal ion chelating agent (see later in the description) which may chelate (trace) metal ions involved in oxidative processes, for example, divalent metal ions involved in enzyme free radical mechanisms leading to apoptosis and cell death. Such metal ions include calcium, iron, zinc and copper. The metal ion chelating agent is preferably hydrophilic in nature. Benefit may also be observed by suitable inhibition of calcium influx induced apoptosis (Ca channel blockers, such as Nifedapine, may also be useful in this context) and/or enhanced hypo-zincaemia and/or a reduction in iron serum levels.

The compositions, methods and uses of the present invention relate to use in warm-blooded animals, in particular such as man, in sedation. Although the compositions are of particular benefit in improving survival in critically ill patients, the compositions may also be used advantageously in other patient populations.

By 'improvement in survival' and 'improving survival' we mean that the critically ill patient is still alive at a clinically significant time, such as 7 days post sedation. A further clinically significant time is 28 days post-sedation. The post-sedation time may be determined from the point at which sedation was commenced (as in the clinical trial programme) or from the point at which sedation was stopped. Also included in these terms is that the critically ill patient demonstrates a reduction, or absence, in condition related problems and/or does not require further mechanical ventiliation and/or is deemed by a physician to be less likely to die and/or is deemed by a physician to have an improved probability of surviving.

The patient populations which benefit the most from the survival aspect of the invention are 'critically ill patients' and are those not receiving sedation for a chronic disease, i.e. 'critically ill patients' are those, for example, suffering an acute insult, injury or trauma and requiring sedation following, for example, (elective or emergency) surgery, medical treatment or post-trauma. In particular, critically ill ventilated patients in ICUs are included. Patients with a probability of dying (e.g. from an underlying disease condition such as severe renal failure) of about 70% or above are less likely to benefit from the survival aspect of the invention. Similarly, a survival benefit from the invention will also be less marked for patients who are intrinsically reasonably healthy, with a probability of dying of about 5% or less. The invention thus provides, in one embodiment, a potential treatment for certain patients in danger of suffering multiple organ dysfunction/failure. In another embodiment the use and method of the invention are provided for surgical patients requiring sedation following elective and/or emergency surgery.

In one aspect of the invention, the components may be administered continuously by infusion (together as a co-infusion or separately) or via intermittent bolus injections. For example, in one embodiment, propofol formulations without a metal ion chelating agent may be administered with an independent administration of a metal ion chelating agent.

The compositions of the present invention are characterised in that they are injectable oil-in-water emulsions in which the oil phase is omega-3 oil rich. Omega-3 oils are polyunsaturated fatty acids, particularly eicosapentaenoic acid (EPA) and docosahexaenoic acid (DHA), or esters thereof such as ethyl or glyceryl esters. EPA is particularly beneficial for immune function response, and DHA is particularly beneficial for neuronal repair.

The oil phase comprises an omega-3 oil (or a mixture of omega-3 oils) and an omega-6 oil (or a mixture of omega-6 oils). The oil phase is present in an amount of up to 30% by weight of the composition, preferably 10-20%.

By an 'omega-3 oil' we mean a water-immiscible lipophilic solvent which is included in the oil phase and is omega-3 rich, such as linseed, fish oils, menhoden, salmon, mackeral, tuna or anchovy-derived oils. Specifically preferred omega-3 oils are DHA and EPA, preferably in a mixture ranging in ratios from DHA:EPA of about 80%:20% to DHA:EPA of about 20%:80%.

By an 'omega-6 oil' we mean a water-immiscible lipophilic solvent which is included in the oil phase and is not omega-3 rich, e.g. vegetable oils such as soy bean, safflower, castor, arachidis, olive, corn, sunflower or cottonseed oil; fractionated oils such as medium chain triglycerides; fractionated coconut oil or modified soy bean oil; long and medium chain fatty acids or fatty acid alcohols, for example a mono-, di-, or triglyceride; or is a chemically modified or manufactured material such as ethyl oleate, isopropyl myristate, isopropyl palmitate, a glycerol ester or polyoxyl hydrogenated castor oil. Specifically preferred omega-6 oils are vegetable oils such as soy bean.

By 'an omega-3 rich water-immiscible solvent' and 'an omega-3 water-immiscible solvent' we mean a water immiscible solvent comprising an omega-3 oil or oils, and an omega-6 oil or oils. The omega-3 oil or oils, and the omega-6 oil or oils are present in a ratio of omega-3 : omega-6 of about 80% : 20% to about 20% : 80%, preferably 50% : 50%.

Thus, typical 'omega-3 rich water-immiscible solvents' comprise
(i) omega-3 = 50% (DHA:EPA = 80% : 20%) : omega-6 (e.g. soy bean oil) = 50%;
(ii) omega-3 = 50% (DHA:EPA = 50% : 50%) : omega-6 (e.g. soy bean oil) = 50%;
(iii) omega-3 = 50% (DHA:EPA = 20% : 80%) : omega-6 (e.g. soy bean oil) = 50%;
(iv) omega-3 = 80% (DHA:EPA = 50% : 50%) : omega-6 (e.g. soy bean oil) = 20%;
(v) omega-3 = 20% (DHA:EPA = 50% : 50%) : omega-6 (e.g. soy bean oil) = 80%.

In a further aspect of the invention pure omega-3 oil may be used.

By an 'oil-in-water emulsion' we mean a distinct two-phase system that is in equilibrium and in effect, as a whole, is kinetically stable and thermodynamically unstable.

By the term 'edetate' we include metal ion chelating/sequestering agents, such as polyaminocarboxylate chelators, such as 'edetate' (ethylenediaminetetraacetic acid -EDTA), diethylenetriaminepentaacetic acid (DTPA) and EGTA, and derivatives thereof. For example, the disodium derivative of edetate is known as disodium edetate. In general, suitable metal ion chelating agents are those salts having lower affinity for the free acid form than calcium, and in particular those derivatives descibed in UK Patent No. 2,298,789. A particular, preferred metal ion chelating agent is disodium edetate. Desferroxime is a further suitable metal ion chelating agent.

In propofol compositions containing edetate, typically the metal ion chelating agent will be present in the compositions in a molar concentration (with respect to the metal ion chelating agent free acid) in the range 3x10⁻⁵ to 9x10⁻⁴. Preferably the metal ion chelating agent free acid is present in the range 3x10⁻⁵ to 7.5x10⁻⁴, for example in the range 5x10⁻⁵ to 5x10⁻⁴ and more preferably in the range 1.5x10⁻⁴ to 3.0x10⁻⁴, most preferably about 1.5x10⁻⁴. In particular, the metal ion chelating agent free acid is present in the range from about 0.0005% to 0.1% w/v. The 0.005 % concentration of disodium edetate used in modified 'Diprivan' was selected to prevent significant growth of microorganisms for at least 24 hours in the event of adventitious, extrinsic contamination. Depending upon the properties of the metal ion chelating agent selected, the benefit of the present invention may be achieved using lower than a 0.005 % concentration.

A propofol composition suitable for use according to the present invention typically comprises from 0.1 to 5%, by weight, of propofol. Preferably the composition comprises from 1 to 2% by weight of propofol and, in particular, about 1% or about 2%. Propofol alone may be emulsified with water by means of a surfactant, but it is preferred that propofol is dissolved in an omega-3 rich water-immiscible solvent prior to emulsification. The omega-3 rich water-immiscible solvent is suitably present in an amount that is up to 30% by weight of the composition, more suitably 5-25%, preferably 10-20% and in particular about 10%.

The oil phase, with or without propofol, is emulsified in an aqueous phase by means of a surfactant. Suitable surfactants include, but are not limited to, phospholipids, e.g. naturally occurring phospholipids such as egg and soy lecithin; synthetic or semisynthetic phospholipids such as phosphatidylcholines, phosphatidylethanolamines and phosphatidylglycerols; ethoxylated phospholipids; glycolipids; sterols such as cholesterol and cholesterol esters; synthetic non-ionic surfactants such as ethoxylated ethers and esters, and polypropylene-polyethylene block copolymers such as poloxamers and poloxamines; or mixtures thereof. Preferred surfactants are phospholipids or mixtures of phospholipids and one or more of the above surfactants.

The compositions may optionally contain other pharmaceutically acceptable agents, e.g. tonicity modifiers such as glycerol and xylitol; pH adjusting agents such as sodium hydroxide and hydrochloric acid; antimicrobial agents, in particular EDTA, chelating agents; or antioxidants such as tocopherols.

The compositions are typically sterile formulations and are prepared according to conventional manufacturing techniques using e.g. aseptic manufacture or terminal sterilisation by autoclaving. Emulsions of predominantly submicron droplet size are obtained by conventional high energy dispersion techniques such as high pressure homogenisation and microfluidisation. The mean droplet diameter is typically in the range of 50 to 1000 nm, particularly between 200 and 500 nm.

Propofol, either alone or dissolved in an omega-3 rich water-immiscible solvent, is emulsified by means of a surfactant. Suitable surfactants include synthetic non-ionic surfactants, for example ethoxylated ethers and esters and polypropylene-polyethylene block co-polymers, and phosphatides for example naturally occuring phosphatides such as egg and soya phosphatides and modified or artificially manipulated phosphatides (for example prepared by physical fractionation and/or chromatography), or mixtures thereof. Preferred surfactants are egg and soya phosphatides.

The compositions suitable for use in the present invention are suitably formulated to be at physiologically neutral pH, typically in the range 6.0-8.5, if necessary by means of alkali such as sodium hydroxide.

The compositions suitable for use in the present invention may be made isotonic with blood by the incorporation of a suitable tonicity modifier for example glycerol.

The compositions suitable for use in the present invention are typically sterile formulations and are prepared according to conventional manufacturing techniques using for example aseptic manufacture or terminal sterilisation by autoclaving. Further details on the preparation of compositions suitable for use in the present invention are included in the Patents referred to herein in the introduction, and are hereby incorporated by reference. Also included are the compositions obtainable by the procedures described in the accompanying Examples.

The compositions suitable for use in the present invention are useful as anaesthetics, which includes sedation and induction and maintenance of general anaesthesia, and such properties may be usefully exploited during the improvement in survival in critically ill patients according to the present invention. Propofol is a short-acting anaesthetic, suitable for both induction and maintenance of general anaesthesia, for sedation to supplement regional analgesic techniques, for sedation of ventilated patients receiving intensive care and for conscious sedation for surgical and diagnostic procedures in Intensive Care Units. Propofol may be administered by single or repeated intravenous bolus injections or by continuous infusion. It is very rapidly removed from the blood stream and metabolised. Thus the depth of sedation is easily controlled and patient recovery on discontinuing the drug is usually rapid and the patient is often significantly more clear headed as compared to after administration of other anaesthetics.

The dosage levels suitable for use according to the invention are generally within those typically used (see the literature available on propofol, for example the dosing information provided in US 5,714,520 column 6, which is hereby incorporated by reference). For 'Diprivan', dose levels in the range 0.3-8.0 mg/Kg/hr for adult humans may be used, but may be optimised to achieve the desired effect in any particular patient, in accordance with normal skill in the art. A continuous infusion at about 0.3-4.0 mg/kg/hr for sub- to moderate sedation is typically used.

In use, the propofol composition of the present invention may be administered for longer than is used for simple sedation, i.e. a patient may thus be maintained under sedation until it is considered that effective treatment has been delivered. Artificial ventilation requires sedation, and the present propofol formulations can be used for this purpose. Simultaneously, the present propofol formulations can improve survival potential by a mechanism that is independent of the artificial ventilation.

The precise nature of the 'omega-3 rich water-immiscible solvent' chosen may be selected to suit a particular patient population.

Certain advantages referred to above for omega-3 rich propofol compositions apply also to omega-3 rich intravenous fat emulsions which may be administered, to patients in need thereof, over periods of a day or more. Intravenous fat emulsions (also known as parenteral, nutrition emulsions) are administered, usually by infusion, to patients having requirements for additional calories and adequate nutrition, by oral or other means, is not desirable or is not possible. Intravenous fat emulsions typically maintain a positive nitrogen balance and provide which optionally comprises an amount of edetate sufficient to prevent significant growth of microorganisms for at least 24 hours (see the concentrations of edetate referred to herein). In particular the present invention provides a sterile, aqueous composition for parenteral administration which comprises an oil-in-water emulsion in which an omega-3 rich water-immiscible solvent is emulsified with water and stabilised by means of a surfactant and which further comprises an amount of edetate (in particular disodium edetate) sufficient to prevent significant growth of microorganisms for at least 24 hours.

### Combination with other therapeutic agents

As a further feature of the present invention there are provided sterile pharmaceutical compositions and emulsions as described herein and which further comprise a therapeutic or pharmaceutical agent.

Suitable therapeutic or pharmaceutical agents are those capable of being administered parenterally in an oil-in-water emulsion. Typically such agents (which may be administered separately, sequentially or simultaneously with the propofol composition) are lipophilic compounds and may for example be antifungal agents, anaesthetics, antibacterial agents, anticancer agents, anti-emetics, antioxidants, agents acting on the central nervous system such as diazepam, steroids, barbiturates and vitamin preparations. The agents most useful are those which may have additional benefit in the treatment or prevention of multiple organ dsyfunction and death and its causes and/or provide an improvement in the immune function, for example, antibacterial agents, anti-inflammatory agents, NSAIDs, Vitamin C, Vitamin E, fluid therapy, vasoactive amines, anti-mitotic agents, amino acids (such as arginine) and RNA. Supportive treatment of organ insufficiency may include artificial ventilation and dialysis.

Thus, there is also provided the use of a pharmaceutical compositions as described herein and which further comprise a therapeutic or pharmaceutical agent, for the manufacture of a medicament for producing a sedative and/or anaesthetic effect (and, in another embodiment for improving survival in critically ill patients). Also provided is a method of improving survival in critically ill patients comprising the use of such compositions. In particular this feature of the present invention relates to such oil-in-water emulsions which typically are administered, to patients in need thereof, over periods of a day or more.

Comments herein relating to typical and preferred propofol compositions for use in the present invention and the preparation thereof apply mutatis mutandis to oil-in-water emulsions containing an additional therapeutic or pharmaceutical agent.

In a further aspect of the invention, there is provided a pharmaceutical composition, method and use substantially as described herein with reference to the description and Examples.

### EXPERIMENTAL

Propofol compositions containing omega-3 oils may be prepared by analagous procedures to those described in US 5,714,520 (the relevant experimental sections of which are incorporated herein by reference).

### Example 1

An aqueous phase is prepared from glycerol (2.25% by weight), disodium edetate (0.005% by weight) and Water for Injection. The mixture is heated to a temperature of approximately 80°C. Egg lecithin (1.2% by weight) is dispersed in the mixture by ultra-turrax vortexing for approximately 5 min. The mixture is cooled down to room temperature, and pH is adjusted to pH 9-10. Omega-3 oil (10% by weight) containing approximately 20% by weight of EPA and 40% by weight of DHA is added to the aqueous phase, and a crude emulsion is prepared by ultra-turrax vortexing at room temperature. The crude emulsion is transferred to a high pressure homogeniser and is circulated through the homogeniser until the desired droplet size is obtained. The emulsion is thermostatised at room temperature during homogenisation. The omega-3 emulsion is sterilised by autoclavation or sterile filtration. All operations are carried out under nitrogen.

The omega-3 emulsion can be mixed or co-infused with a commercially available propofol emulsion such as Diprivan-EDTA (e.g. 1% or 2%), preferably in a ratio of 1:4 to 4:1, in particular 1:1, to give an omega-3 rich propofol formulation.

### Example 2

An aqueous phase is prepared from glycerol (2.25% by weight), disodium edetate (0.005% by weight) and Water for Injection. The mixture is heated to a temperature of approximately 80°C. Egg lecithin (1.2% by weight) is dispersed in the mixture by ultra-turrax vortexing for approximately 5 min. The mixture is cooled down to room temperature, and pH is adjusted to pH 9-10. Omega-3 oil (10% by weight) containing approximately 40% by weight of EPA and 20% by weight of DHA is added to the aqueous phase, and a crude emulsion is prepared by ultra-turrax vortexing at room temperature. The crude emulsion is transferred to a high pressure homogeniser and is circulated through the homogeniser until the desired droplet size is obtained. The emulsion is thermostatised at room temperature during homogenisation. The omega-3 emulsion is sterilised by autoclavation or sterile filtration. All operations are carried out under nitrogen.

The omega-3 emulsion can be mixed or co-infused with a commercially available propofol emulsion such as Diprivan EDTA (e.g. 1% or 2%), preferably in a ratio of 1:4 to 4:1, in particular 1:1, to give an omega-3 rich propofol formulation.

### Example 3 - Omega-3 emulsion containing propofol

An aqueous phase is prepared from glycerol (2.25% by weight), disodium edetate (0.005% by weight) and Water for Injection. The mixture is heated to a temperature of approximately 80°C. Egg lecithin (1.2% by weight) is dispersed in the mixture by ultra-turrax vortexing for approximately 5 min. The mixture is cooled down to room temperature, and pH is adjusted to pH 9-10. Omega-3 oil (5% by weight), soy bean oil (5% by weight) and propofol (1% by weight) are mixed and added to the aqueous phase. A crude emulsion is prepared by ultra-turrax vortexing at room temperature. The crude emulsion is transferred to a high pressure homogeniser and is circulated through the homogeniser until the desired droplet size (such as a mean of 250nm) is obtained. The emulsion is thermostatised at room temperature during homogenisation. The propofol containing omega-3 emulsion is sterilised by autoclavation or sterile filtration. All operations are carried out under nitrogen.

Survival benefit may be assessed in various animal model studies such as, a rat sepsis model (using endotoxin), a rat haemorrhage model, diffuse brain injury or head trauma model. Such models are conducetd according to standard procedures well known in the art. Standard 'Diprivan' may be used as a comparator to assesss the influence of the omega-3 lipid. An infusion rate of about 2 to 3 ml/hr (for example by co-infusion via a Y-piece of a suitable omega-3 emulsion and a commercially available propofol emulsion) may be used. Survival may be noted at a suitable time interval, for example after 24 hours.

## Claims

1. A sterile pharmaceutical composition for parenteral administration which comprises an oil-in-water emulsion in which a free radical scavenging sedative agent dissolved in an omega-3 rich water-immiscible solvent, which is a mixture of an omega-3 oil or oils and an omega-6 oil or oils in the ratio of omega-3 : omega-6 of from 80% : 20% to 20% : 80%, and a metal ion chelating agent, is emulsified with water and stabilised by means of a surfactant, the composition being suitable either directly or after dilution with a liquid diluent for parenteral administration to a warm-blooded animal.

2. A sterile pharmaceutical composition for parenteral administration according to claim 1, in which the free radical scavenging sedative agent is propofol.

3. A sterile pharmaceutical composition according to any one of claims 1 to 2, in which the metal ion chelating agent is edetate.

4. A sterile pharmaceutical composition according to any one of claims 1 to 3, which comprises up to about 30% by weight of omega-3 rich water-immiscible solvent

5. A sterile pharmaceutical composition according to any one of claims 1 to 4, which comprises from about 10% to about 20% by weight of omega-3 rich water-immiscible solvent.

6. A sterile pharmaceutical composition according to any one of claims 1 to 5, wherein the omega-3 oil is eicosapentaenoic acid (EPA), docosahexaenoic acid (DHA) or a mixture of EPA and DHA,

7. A sterile pharmaceutical composition according to claims 1 to 6, wherein the omega-6 oil is soy bean oil.

8. A sterile pharmaceutical composition according to any one of claims 1 to 7, wherein the surfactant is a naturally occurring phosphatide.

9. A sterile pharmaceutical composition according to claim 8, wherein the phosphatide is egg phosphatide or soya phosphatide.

10. A sterile pharmaceutical composition according to any one of claims 2 to 9 which comprises from about 1% to about 2% by weight of propofol.

11. A sterile pharmaceutical composition in the form of an oil-in-water emulsion which comprises:
(a) 1% by weight of propofol,
(b) 10% by weight of omega-3 rich water-immiscible solvent, which is a mixture of an omega-3 oil or oils and an omega-6 oil or oils in the ratio of omega-3 : omega-6 of from 80% : 20% to 20% : 80%,
(c) 1.2% by weight of egg phosphatide,
(d) 2.25% by weight of glycerol,
(e) sodium hydroxide,
(f) water,
(g) edetate.

12. A sterile pharmaceutical composition in the form of an oil-in-water emulsion which comprises:
(a) 2% by weight of propofol,
(b) 10% by weight of omega-3 rich water-immiscible solvent, which is a mixture of an omega-3 oil or oils and an omega-6 oil or oils in the ratio of omega-3 : omega-6 of from 80% : 20% to 20% : 80%,
(c) 1.2% by weight of egg phosphatide,
(d) 2.25% by weight of glycerol,
(e) sodium hydroxide,
(f) water,
(g) edetate.

13. A sterile pharmaceutical composition according to claim 11 or 12, in which the sterile pharmaceutical composition contains 0.005% by weight of disodium edetate.

## Patentansprüche

1. Sterile pharmazeutische Zusammensetzung zur parenteralen Verabreichung, enthaltend eine Öl-in-Wasser-Emulsion, in der ein Beruhigungsmittel mit radikalfangender Wirkung gelöst in einem an Omega-3 reichen, nicht mit Wasser mischbaren Lösungsmittel, bei dem es sich um eine Mischung eines oder mehrerer Omega-3-Öle und eines oder mehrerer Omega-6-Öle im Verhältnis Omega-3:Omega-6 von 80%:20% bis 20%:80% handelt und ein metallionenchelatisierendes Mittel emulgiert mit Wasser und stabilisiert durch ein Tensid vorliegen, wobei die Zusammensetzung entweder direkt oder nach Verdünnen mit einem flüssigen Verdünnungsmittel für die parenterale Verabreichung an einen Warmblüter geeignet ist.

2. Sterile pharmazeutische Zusammensetzung zur parenteralen Verabreichung nach Anspruch 1, wobei es sich bei dem Beruhigungsmittel mit radikalfangender Wirkung um Propofol handelt.

3. Sterile pharmazeutische Zusammensetzung nach Anspruch 1 oder 2, wobei es sich bei dem metallionenchelatisierenden Mittel um Edetat handelt.

4. Sterile pharmazeutische Zusammensetzung nach einem der Ansprüche 1 bis 3, enthaltend bis zu etwa 30 Gew.-% an Omega-3-reichem, nicht mit Wasser mischbarem Lösungsmittel.

5. Sterile pharmazeutische Zusammensetzung nach einem der Ansprüche 1 bis 4, enthaltend etwa 10 Gew.-% bis etwa 20 Gew.-% an Omega-3-reichem, nicht mit Wasser mischbarem Lösungsmittel.

6. Sterile pharmazeutische Zusammensetzung nach einem der Ansprüche 1 bis 5, wobei es sich bei dem Omega-3-Öl um Eicosapentaensäure (EPA), Docosahexaensäure (DHA) oder eine Mischung von EPA und DHA handelt

7. Sterile pharmazeutische Zusammensetzung nach einem der Ansprüche 1 bis 6, wobei es sich bei dem Omega-6-Öl um Sojabohnenöl handelt.

8. Sterile pharmazeutische Zusammensetzung nach einem der Ansprüche 1 bis 7, wobei es sich bei dem Tensid um ein natürliches Phosphatid handelt.

9. Sterile pharmazeutische Zusammensetzung nach Anspruch 8, wobei es sich bei dem Phosphatid um Eiphosphatid oder Sojaphosphatid handelt.

10. Sterile pharmazeutische Zusammensetzung nach einem der Ansprüche 2 bis 9, enthaltend etwa 1 Gew.-% bis etwa 2 Gew.-% an Propofol.

11. Sterile pharmazeutische Zusammensetzung in Form einer Öl-in-Wasser-Emulsion, enthaltend:
(a) 1 Gew.-% an Propofol
(b) 10 Gew.-% an einem an Omega-3 reichen, nicht mit Wasser mischbaren Lösungsmittel, bei dem es sich um eine Mischung eines oder mehrerer Omega-3-Öle und eines oder mehrerer Omega-6-Öle im Verhältnis Omega-3:Omega-6 von 80%:20% bis 20%:80% handelt
(c) 1,2 Gew.-% an Eiphosphatid
(d) 2,25 Gew.-% an Glycerin
(e) Natriumhydroxid
(f) Wasser
(g) Edetat

12. Sterile pharmazeutische Zusammensetzung in Form einer Öl-in-Wasser-Emulsion, enthaltend:
(a) 2 Gew.-% an Propofol
(b) 10 Gew.-% an einem an Omega-3 reichen, nicht mit Wasser mischbaren Lösungsmittel, bei dem es sich um eine Mischung eines oder mehrerer Omega-3-Öle und eines oder mehrerer Omega-6-Öle im Verhältnis Omega-3:Omega-6 von 80%:20% bis 20%:80% handelt
(c) 1,2 Gew.-% an Eiphosphatid
(d) 2,25 Gew.-% an Glycerin
(e) Natriumhydroxid
(f) Wasser
(g) Edetat

13. Sterile pharmazeutische Zusammensetzung nach Anspruch 11 oder 12, wobei die sterile pharmazeutische Zusammensetzung 0,005 Gew.-% an Dinatriumedetat enthält.

## Revendications

1. Composition pharmaceutique stérile destinée à une administration par voie parentérale, comprenant une émulsion huile-dans-eau dans laquelle un agent sédatif anti-radicalaire solubilisé dans un solvant non miscible à l'eau et riche en oméga-3, qui est un mélange d'une huile ou d'huiles oméga-3 et d'une huile ou d'huiles oméga-6 selon un rapport oméga-3 : oméga-6 allant de 80% : 20% à 20% : 80%, et d'un agent chélateur d'ions métalliques, est émulsifié par de l'eau et stabilisé au moyen d'un agent tensioactif, la composition étant convenable soit directement, soit après une dilution par un diluant liquide pour une administration par voie parentérale à un animal à sang chaud.

2. Composition pharmaceutique stérile destinée à une administration par voie parentérale selon la revendication 1, dans laquelle l'agent sédatif anti-radicalaire est le propofol.

3. Composition pharmaceutique stérile selon l'une quelconque des revendications 1 à 2, dans laquelle l'agent chélateur d'ions métalliques est l'édétate.

4. Composition pharmaceutique stérile selon l'une quelconque des revendications 1 à 3, comprenant jusqu'à environ 30% en poids de solvant non miscible à l'eau et riche en oméga-3.

5. Composition pharmaceutique stérile selon l'une quelconque des revendications 1 à 4, comprenant d'environ 10% à environ 20% en poids de solvant non miscible à l'eau et riche en oméga-3.

6. Composition pharmaceutique stérile selon l'une quelconque des revendications 1 à 5, dans laquelle l'huile oméga-3 est l'acide éicosapentaénoïque (EPA), l'acide docosahexaénoïque (DHA) ou un mélange d'EPA et de DHA.

7. Composition pharmaceutique stérile selon les revendications 1 à 6, dans laquelle l'huile oméga-6 est l'huile de soja.

8. Composition pharmaceutique stérile selon l'une quelconque des revendications 1 à 7, dans laquelle l'agent tensioactif est un phospholipide présent naturellement.

9. Composition pharmaceutique stérile selon la revendication 8, dans laquelle le phospholipide est le phospholipide d'oeuf ou le phospholipide de soja.

10. Composition pharmaceutique stérile selon l'une quelconque des revendications 2 à 9, comprenant d'environ 1% à environ 2% en poids de propofol.

11. Composition pharmaceutique stérile sous forme d'une émulsion huile-dans-eau, comprenant :
(a) 1% en poids de propofol,
(b) 10% en poids de solvant non miscible à l'eau et riche en oméga-3, qui est un mélange d'une huile ou d'huiles oméga-3 et d'une huile ou d'huiles oméga-6 selon un rapport oméga-3 : oméga-6 allant de 80% : 20% à 20% : 80%,
(c) 1,2% en poids de phospholipide d'oeuf,
(d) 2,25% en poids de glycérol,
(e) de l'hydroxyde de sodium,
(f) de l'eau,
(g) de l'édétate.

12. Composition pharmaceutique stérile sous forme d'une émulsion huile-dans-eau, comprenant :
(a) 2% en poids de propofol,
(b) 10% en poids de solvant non miscible à l'eau et riche en oméga-3, qui est un mélange d'une huile ou d'huiles oméga-3 et d'une huile ou d'huiles oméga-6 selon un rapport oméga-3 : oméga-6 allant de 80% : 20% à 20% : 80%,
(c) 1,2% en poids de phospholipide d'oeuf,
(d) 2,25% en poids de glycérol,
(e) de l'hydroxyde de sodium,
(f) de l'eau,
(g) de l'édétate.

13. Composition pharmaceutique stérile selon la revendication 11 ou 12, dans laquelle la composition pharmaceutique stérile contient 0,005% en poids d'édétate disodique.
